# EUROPEAN PATENT APPLICATION

(11) **EP 3 804 697 A1**
(43) Date of publication of application: **14.04.2021**
(21) Application number: 20201399.1
(22) Date of filing: 12.10.2020
(51) Int. Cl.: A61K 9/00, A61K 9/20, A61K 31/135, A61K 31/53, A61P 25/04, A61P 29/00

(54) **COMBINATION PHARMACEUTICAL PREPARATION FOR ORAL APPLICATION COMPRISING LAMOTRIGINE AND SERTRALINE, ITS PREPARATION AND USE**

(30) Priority: 12.10.2019 HU 1900358
(71) Applicant: MEDITOP Gyógyszeripari Kft., 2097 Pilisborosjenö (HU)
(72) Inventor: Ács, Zoltán, 2097 Pilisborosjen (HU); Fekete, Pál, 1141 Budapest (HU); Greskovits, Dávid, 2000 Szentendre (HU)
(74) Representative: Kovári, Zoltán

(57) **Abstract**

The object of the invention relates to a combination pharmaceutical preparation for oral administration, which contains lamotrigine and sertraline as active substances, and microcrystalline cellulose as filler in such a way that the particle size of the lamotrigine is D90 = 50 µm, the particle size of the sertraline is D90 = 50 µm and the particle size of the microcrystalline cellulose is D10 = 50 µm. The pharmaceutical preparation according to the invention is preferably for the treatment of pain. The object of the invention also relates to a method for the production of the pharmaceutical preparation.

## Description

### The field of the invention

The object of the present invention relates to a solid pharmaceutical preparation containing the active ingredients lamotrigine and sertraline, to its preparation and to the use of such pharmaceutical preparation.

### The state of the art

Lamotrigine belongs to the group of anti-epileptic active substances. It is currently used in the treatment of epilepsy and bipolar depression. The chemical name of lamotrigine is 3,5 -diamino-6-(2,3-dichlorophenyl)-1,2,4-triazine, its structural formula is the following:

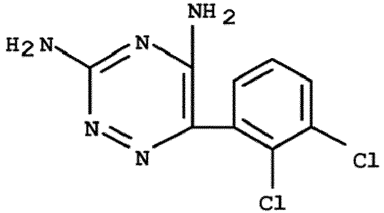

Sertraline is one of the selective serotonine reuptake inhibitors (SSRIs). Active substances of this type are used in the treatment of depression and anxiety conditions. The chemical name of sertraline is (1S,4S)-4-(3,4-dichlorophenyl)-N-methyl-1,2,3,4-tetrahydronaphthalen-1-amine hydrochloride, its structural formula is the following:

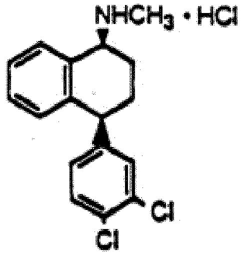

The active substance lamotrigine was developed by the GlaxoSmithKline company and was placed on the market in the United Kingdom in 1991 under the brand name Lamictal. The tablets contain the excipients of lactose monohydrate, microcrystalline cellulose, Povidone K30, sodium carboxymethyl starch, yellow iron oxide, and magnesium stearate (OGYI-T 4094, 25.08.2011).
The active substance suffers significant degradation in acidic, alkaline and oxidative conditions, and it is particularly sensitive to alkaline degradation. Moisture, light and heat have no effect on the stability of lamotrigine (Journal of Chemistry, Volume 2013, Article ID 608196: Stress Degradation Assessment of Lamotrigine Using a Validated Stability-Indicating HPTLC Method, Karim Michail et al.). On the basis of the composition of the preparation, before the tablets are produced the active substance and the excipients are granulated in the aqueous solution of Povidone and then dried.

The active substance sertraline was developed by the Pfizer company and was placed on the market in the United States of America in 1991 under the brand name Zoloft. The tablets contain the excipients of lactose monohydrate, microcrystalline cellulose, Povidone K30, cross-linked carboxymethyl cellulose sodium, magnesium stearate and also contain a hydroxypropyl methylcellulose-based film coating. The active substance suffers significant degradation in oxidative conditions (International Journal of Pharmaceutics, Volume 461, 30 January 2014, Pages 322-330). On the basis of the compositions of the preparation, before the tablets are produced the active substance and the excipients are granulated using wet or dry granulation.

European patent number EP2983787B1 mentions - among others - lamotrigine and sertraline as an antidepressant as the second possible active substance beside an active substance for treating post-traumatic stress disorder.

European patent number EP3010914B1 mentions sertraline as an antidepressant as a further possible active substance of a combination pharmaceutical preparation and lamotrigine as a mood stabiliser in a different approach.

European patent number EP2542227B1 relates to a tablet that first provides immediate active substance release and then delayed active substance release. It mentions several hundreds possible active substances that this tablet may be used in connection with, it also mentions lamotrigine and sertraline among the possible central nervous system active substances.

European patent number EP2175728 B1 relates to medications for treating pain with sodium channel blockers. It mentions numerous active substances as those worthwhile combining with the sodium channel blocker, sertraline is also mentioned among these as an SSRI. They write in general about its tableting options.

Hungarian patent registration number HU230403B1 (and European patent number EP1699489 B1 belonging to the same patent family) discloses an active substance combination where the selective SSRI active substance is sertraline and the selective sodium channel blocker is lamotrigine. Among other applications this combination pharmaceutical preparation is suitable for treating chronic pain. Among the possible formulations it mentions tablets and lists the usual tableting excipients, among which it mentions microcrystalline cellulose among the possible fillers.

The difficulties of the production of pharmaceutical preparations containing these active substances lie in that the various polymorphs of sertraline have varying stability and so demand differing formulation approaches, and in order to achieve a quick effect it is necessary for both active substances to dissolve jointly and quickly.

### A brief description of the invention

During our investigations it was surprising to experience that if lamotrigine and sertraline of the appropriate particle size are formulated with microcrystalline cellulose of the appropriate particle size into an oral pharmaceutical preparation, then the above problems may be overcome.

In other words, the present invention relates to a combination pharmaceutical preparation for oral administration and that contains lamotrigine and sertraline as active substances, and furthermore contains microcrystalline cellulose as the filler in such a way that the particle size of lamotrigine is D90 = 50 µm, the particle size of sertraline is D90 = 50 µm, and the particle size of the microcrystalline cellulose is D10 = 50 µm.

According to a preferred embodiment of the combination pharmaceutical preparation for oral administration according to the present invention, it also contains one or more of the following as excipients: disintegrant, lubricant, glidant.

An even more preferred embodiment of the combination pharmaceutical preparation for oral administration according to the present invention contains sodium carboxymethyl starch as disintegrant.

According to another preferred embodiment of the combination pharmaceutical preparation for oral administration according to the present invention it contains excipients selected from the group containing the following: sodium carboxymethyl starch, magnesium stearate and colloidal silicon dioxide.

According to a preferred embodiment of the combination pharmaceutical preparation for oral administration according to the present invention, it is a tablet or a capsule, preferably a tablet. According to the most preferred embodiment of the present invention, the combination pharmaceutical preparation for oral administration is a tablet containing a film coating.

The object of the present invention also relates to the use of the above combination pharmaceutical preparation for oral administration for the treatment of chronic pain. In this indication the tablet preferably has a mass of 300 mg and one tablet contains 30 mg lamotrigine and 15 mg sertraline (in the form of a base).

The object of the present invention also relates to a method for the production of the above combination pharmaceutical preparation for oral administration, during which method lamotrigine with a particle size of D90 = 50 µm, sertraline with a particle size of D90 = 50 µm and microcrystalline cellulose with a particle size of D10 = 50 µm are mixed, optionally with other excipients, and formed into tablets or filled into capsules.

### A detailed description of the invention

During investigations aimed at realising the above objectives it was found in a way surprising to a person skilled in the art that with the use of a minimal number of excipients the requirements of pharmaceutical preparations may be achieved in the case of the combination pharmaceutical preparation for oral administration forming the object of the present invention, i.e. evenly distributed active substance content, appropriate chemical stability and appropriate *in vitro* dissolution as long as the particle size of the active substances (lamotrigine and sertraline) is D90 = 50 µm and the particle size of the microcrystalline cellulose used as filler is D10 = 50 µm.

The small particle size of the active substances ensures, on the one part, suitable dissolution. On the other part, however, as is known to a person skilled in the art, the powder-flow properties of active substances with a small particle size are unsuitable for tableting, this is why it is generally necessary to granulate them. The present invention is based on the recognition that by using a filler (microcrystalline cellulose in the present case) with a larger particle size next to the small particle size active substances, granulation is not necessary in order to create a solid oral pharmaceutical form.

In other words, the basis of the present invention is that the small particle size lamotrigine and sertraline with the use of larger particle size microcrystalline cellulose may be formulated into a combination pharmaceutical preparation for oral administration, such as a tablet or capsule, without granulation.

In the scope of the present invention the particle size "D90 = 50 µm" means that 90% by volume of the particles are 50 µm or smaller, i.e. their D90 value is 50 µm. On the other part, the particle size "D10 = 50 µm" means that 90% by volume of the particles are larger than 50 µm, i.e. their D10 value is 50 µm.

According to investigations the 50 µm upper limit of particle size in the case of the active substances and, in addition, the 50 µm lower limit of particle size in the case of the filler microcrystalline cellulose together ensure that a combination pharmaceutical preparation for oral administration containing lamotrigine and sertraline disintegrates suitable quickly and thereby ensures the desired active substance release.

The reason for this is that the combination pharmaceutical preparation for oral administration according to the present invention may be used in indications in the case of which fast active substance release is important. Such indications include, for example, pain, particularly chronic pain. Naturally, the pharmaceutical preparation according to the present invention may also be used in other indications where the combination of lamotrigine and sertraline may be brought into consideration as active substance combination.

In addition to the necessary microcrystalline cellulose as filler, the pharmaceutical preparation for oral administration according to the present invention may also optionally contain further excipients. Such further excipients known to a person skilled in the art are disintegrants, lubricants and glidants. The knowledge relating to the type of excipients, their use and other characteristics is available to a person skilled in the art in the well known handbooks, such as in the following: Handbook of Pharmaceutical Excipients, 6th Edition; 2009; Published by the Pharmaceutical Press.

The use of disintegrants (materials facilitating the breaking down of materials), lubricants (materials facilitating sliding) and glidants (materials improving flowability) in the case of the combination pharmaceutical preparation for oral administration according to the present invention is not absolutely necessary, however, it may be preferable in order to improve specific characteristics of the pharmaceutical preparation.

The customary excipients may be used as disintegrant and lubricant in the combination pharmaceutical preparation for oral administration according to the present invention, such as sodium carboxymethyl starch, carboxymethyl cellulose, crospovidone, magnesium stearate, and stearic acid. Colloidal silicon dioxide may also be used to improve the flowability of the powder mixture serving as the basis of the pharmaceutical preparation.

The combination pharmaceutical preparation for oral administration according to the present invention is preferably a tablet or capsule, even more preferably a tablet. The tablets and capsules quickly disintegrate in an aqueous medium, i.e. after swallowing the medication, and it is this that ensures the quick dissolution of the active substances.

One of the most preferred embodiments of the present invention is when the combination pharmaceutical preparation for oral administration is a tablet with a film coating. Such a tablet according to the present invention has appropriate strength. The production of tablets, capsules and film-coated tablets is well known to a person skilled in the art. Nevertheless, reference is made in this respect to the Remington handbook (Remington's Pharmaceutical Sciences, Gennaro, A. R. (editor), 18th edition, Easton, PA, Mack Publishing Co. (1990)).

The combination pharmaceutical preparation for oral administration according to the present invention serves for the treatment of pain, particularly preferably chronic pain. It is known according to the state of the art that the two active substances, lamotrigine and sertraline are suitable for treating pain, particularly chronic pain. If according to a preferred embodiment of the invention the pharmaceutical preparation is a tablet, then its active substance composition may be, for example, 30 mg lamotrigine and 15 mg sertraline (in the form of a base) in the case of a tablet with a mass of 300 mg.

The object of the present invention also relates to a method for the production of the above combination pharmaceutical preparation for oral administration, during which method lamotrigine with a particle size of D90 = 50 µm, sertraline with a particle size of D90 = 50 µm and microcrystalline cellulose with a particle size of D10 = 50 µm, and, optionally, other excipients are mixed and formed into tablets or filled into capsules. Such formulation methods are well known to a person skilled in the art, reference is made here to the aforementioned Remington handbook.

Neither wetting nor drying steps are used during the method for the production of the combination pharmaceutical preparation for oral administration according to the present invention.

### EXAMPLES

The further details of the invention are disclosed in the following examples without restricting the invention to the examples.

### Example 1: granulation with an aqueous suspension of L-HPC

**Table 1: composition of the tableting mixture**

| Component | Amount (g) |
|---|---|
| Lamotrigine | 15 |
| Sertraline HCl form I | 8.4 |
| Microcrystalline cellulose | 120.6 |
| Sodium carboxymethyl starch | 4 |
| Magnesium stearate | 1 |
| Colloidal silicon dioxide | 1 |
| Total | 150 |

The particle size of the active substances lamotrigine and sertraline is D90 = 50 µm and the particle size of microcrystalline cellulose is D10 = 50 µm.

The active substances according to the above table, the sodium carboxymethyl starch and the colloidal silicon dioxide are mixed together, sieved through a 0.8 mm sieve, filled into the vessel of a mixing apparatus, then the microcrystalline cellulose is added and then this is mixed for 5 minutes at 100 rpm. The magnesium stearate is sieved through a 0.8 mm sieve, added to the mixture, which is then mixed for 1 minute at 100 rpm.

Tablets are pressed from the mixture obtained in this way (100 mm lens tool, 300 mg mass tablets, active substance content: 30 mg lamotrigine and 15 mg sertraline (in base form) per tablet).

**Table 2: the pharmaceutical technology parameters of the tablets:**

| Tablet mass (mg) | 296 mg |
|---|---|
| Resistance to crushing (N) Ph. Eur. 9, 2.9.8 | 96 N |
| Disintegration time (s) Ph. Eur. 9, 2.9.1 | 90 |
| Friability* Ph. Eur. 9, 2.9.7 | 0.04% |
| Dissolution (15 minutes) % Ph. Eur. 9, 2.9.3 mixing apparatus, 100 rpm, pH 4.5 buffer | lamotrigine: 87.6% |
| | sertraline: 95.4% |

| | |
|---|---|
| *Friability is the loss of mass of the tablets occurring as a result of 100 drops in an apparatus according to the prescriptions of the pharmacopoeia. The general requirement relating to friability: max. 1%. (Ph. Eur, 9, 2.9.7.) | |

### Example 2:

**Table 3: composition of the tableting mixture**

| Component | Amount (g) |
|---|---|
| Lamotrigine | 4200 |
| Sertraline HCl form I | 2352 |
| Microcrystalline cellulose | 33768 |
| Sodium carboxymethyl starch | 1120 |
| Magnesium stearate | 280 |
| Colloidal silicon dioxide | 280 |
| Total | 42000 |

The active substances according to the above table, the microcrystalline cellulose, the sodium carboxymethyl starch disintegrant and the colloidal silicon dioxide are sieved through a 0.8 mm sieve, filled into the vessel of a mixing apparatus, then mixed for 5 minutes at 100 rpm. The magnesium stearate is sieved through a 0.8 mm sieve, added to the mixture, which is then mixed for 1 minute at 100 rpm.

Tablets are pressed from the mixture obtained in this way (100 mm lens tool, 300 mg mass tablets, active substance content: 30 mg lamotrigine and 15 mg sertraline (in the form of a base) per tablet).

The tablets are provided with a film coating in an O'Hara tablet coating apparatus by spraying the following film coating solution onto the tablets:

**Table 4: film coating**

| Film coating material | Amount (g) |
|---|---|
| Sepicoat white* | 1400 g |
| Purified water** | 5600 g |

| | |
|---|---|
| *The components of Sepicoat white: modified starch, talc, polyethylene glycol, soya lecithin, titanium dioxide. **Purified water: evaporated during production. | |

**Table 5: the quality parameters of the tablets obtained according to the above**

| Tests | Quality requirement | Result |
|---|---|---|
| Appearance | white, round, biconvex film-coated tablet | compliant |
| Dimensions | diameter: 10.0 ± 0.2 mm | 10.03 mm |
| | height: 3.0 - 5.0 mm | 4.02 mm |
| Average mass | 310.0 mg ± 5.0% (294.5 mg - 325.5 mg) | 310.4 mg |
| Individual mass | The mass of a maximum of two film-coated tablets deviates by more than ± 5.0% from the average mass, and not one deviates by more than ± 10.0% | 306.1-315.3 mg |
| Resistance to crushing | at least 150 N | 207 N |
| | | 160-238 N |
| Disintegration | max 15 minutes | 0'54" |
| Drying loss | max 5.0% | 3.63% |
| Dissolution in 30 minutes in 0.01 N HCl | at least 80 (Q) %/30 minutes | 98.98% |
| | at least 80 (Q) %/30 minutes | 95.55% |
| Active substance content | 30.0 mg ± 10.0%/film-coated tablet | 31.10 mg |
| | 15.0 mg ± 10.0%/film-coated tablet | 14.93 mg |
| Related compounds: | | |
| - Greatest individual unknown: | max 0.2% | 0.003% |
| -Total contaminants: | max 0.5% | 0.020% |

Therefore, tablets with good mechanical properties and a very short disintegration time could be produced with the composition according to the invention.

## Claims

1. Combination pharmaceutical preparation for oral administration, which contains lamotrigine and sertraline as active substances, and microcrystalline cellulose as filler in such a way that the particle size of the lamotrigine is D90 = 50 µm, the particle size of the sertraline is D90 = 50 µm and the particle size of the microcrystalline cellulose is D10 = 50 µm.

2. Combination pharmaceutical preparation for oral administration according to claim 1, which contains one or more of the following as excipients: disintegrant, lubricant, glidant.

3. Combination pharmaceutical preparation for oral administration according to claim 2, which contains sodium carboxymethyl starch as disintegrant.

4. Combination pharmaceutical preparation for oral administration according to claim 1, which contains one or more of the following as excipients: sodium carboxymethyl starch, magnesium stearate, colloidal silicon dioxide.

5. Combination pharmaceutical preparation for oral administration according to any of claims 1 to 4, which is a tablet or capsule, preferably a tablet.

6. Combination pharmaceutical preparation for oral administration according to claim 5, which is a tablet containing a film coating.

7. Combination pharmaceutical preparation for oral administration according to any of claims 1 to 6 for use in the treatment of chronic pain.

8. Combination pharmaceutical preparation for oral administration according to claim 7, which is in tablet form, and where the mass of the tablet is 300 mg and one tablet contains 30 mg lamotrigine and 15 mg sertraline (in the form of a base).

9. Method for the production of a combination pharmaceutical preparation for oral administration, during which method lamotrigine with a particle size of D90 = 50 µm, sertraline with a particle size of D90 = 50 µm and microcrystalline cellulose with a particle size of D10 = 50 µm, and, optionally other excipients are mixed and formulated into tablets or filled into capsules.
